# EUROPEAN PATENT APPLICATION

(11) **EP 3 078 740 A1**
(43) Date of publication of application: **12.10.2016**
(21) Application number: 15796410.7
(22) Date of filing: 13.05.2015
(51) Int. Cl.: C12N 7/04, A61L 2/08

(54) **METHOD FOR INACTIVATING, ON MEDICAL INSTRUMENTS, VIRUSES CONTAINING RNA AND DNA, AND APPARATUS FOR IMPLEMENTING SAME**

(30) Priority: 23.05.2014 UZ 1400210
(71) Applicant: Mkrtchyan, Ovik Leonardovich, Tashkent 100187 (UZ)
(72) Inventor: Mkrtchyan, Ovik Leonardovich, Tashkent 100187 (UZ)
(74) Representative: Diehl & Partner GbR
(86) International application number: PCT/UZ2015/000001
(87) International publication number: WO 2015/179880

(57) **Abstract**

*Usage:* the photodynamic inactivation, on medical instruments, of viruses containing RNA and DNA. *Aim:* to develop an effective method, involving minimal outlay of effort and energy, for inactivating, on medical instruments, viruses containing RNA and DNA, and also an apparatus for implementing same by achieving maximal activation of a photochemical agent during interaction with monochromatic light. Essence of the invention: a method for inactivating, on medical instruments, viruses containing RNA and DNA, including the preliminary mechanical cleaning and washing of the instruments, then keeping the instruments in an aqueous solution of methylene blue with maximum activation taking place during the interaction of the solution with monochromatic light. In the method according to the invention, maximum activation is achieved in a solution of methylene blue having a concentration of 0.01-0.02% by means of interaction with light in the emission spectrum of monochromatic emitters having wavelengths ranging between 582 and 592 nm or between 658 and 662 nm and an overall light output of at least 280 lm. The instruments are kept in the solution for 90 minutes. An apparatus for inactivating, on medical instruments, viruses containing RNA and DNA comprises a hermetically-sealing housing with a chamber, an instrument container which is located in the chamber and which is filled with a solution of methylene blue, and, installed above the container, a source of monochromatic light. The apparatus according to the invention contains a source of monochromatic light comprising monochromatic emitters in an emission spectrum having wavelengths ranging between 582 and 592 nm or between 658 and 662 nm and an overall light output of at least 280 Im. LEDs are selected as the monochromatic emitters.

## Description

The invention is related to photodynamic inactivation of RNA and DNA viruses on medical instruments.

During last years of XX century and at the beginning of XXI century there was a pick of viruses related deceases spread in the world, they had a high risk of threat for humankind survival. An essential number of people are infected, especially with hepatitis B (hereinafter - HBV), hepatitis C (hereinafter HCV) mostly by infected with viruses medical instruments (surgical, dental, ophthalmic, gynecologic and others), which cannot be sterilized under high temperature or by autoclaving. HBV and HCV are resistant to many disinfectants used in medicine. A complete removal of viral particles from the surface of medical instruments and mechanical methods are not feasible. Consequently, virus particles capable of replication) and infecting people are stored on the tools. For human infection and disease development existence of even one particle of HBV or HCV in blood is enough. This leads to a high risk of contracting viral infections of patients with medical procedures. Consequently, there is an urgent need to develop the most effective method of inactivation (deprivation of the ability to replicate) RNA and DNA viruses and particles on medical instruments.

It is clear that photodynamic inactivation of DNA and RNA viruses is based on ability of some chemical substances (photosensitizers) in light change to photoactive condition and generate active forms of oxygen. Abovementioned forms of oxygen are highly toxic compounds, which make for photodynamic activity of current method.

There is a method of inactivating viruses in biological fluids by processing a biological fluid with light for the inactivation of contaminants, such as viruses. According to this method light intensity of at least 30mW/cm² which affect the biological fluid is created. Biological fluid includes a certain amount of the photochemical agent (photosensitizer). The interaction of the photochemical agent with a light activated photochemical agent takes place and it provides viral inactivation. A famous method is based on the activation of methylene blue in its interaction with light having an intensity of at least 30 mW/cm². Methylene blue may be disposed within a biological fluid and may interact with the light for a period between approximately 0.3 and 30 minutes. According to the invention, the use of known high intensity light with a biological fluid such as blood or blood plasma containing a predetermined amount of methylene blue enhances the effect of methylene blue in killing viruses. Methylene blue is activated by high intensity light having wavelengths from about 550 nm to 700 mn, with a peak at 663 nm. The light absorption in this range provides the activation of methylene blue. To create a high-intensity light used high pressure sodium lamps [Description to patent RU Nº 2219951, IPC A61L2 / 08, A61M1 / 36, A01N1 / 02, H01J37 / 20, publ. 27.12.2003].

However, the use of a known method for inactivating RNA and DNA containing viruses on instruments is not effective because of the high-energy intensity, the structural complexity and laboriousness operation.

The closest to the essential features claimed can be a known method of installation for the inactivation of RNA and DNA viruses for medical instruments. In accordance with a method medical instruments (dental, gynecological, surgery, ophthalmic, etc.) after use is subjected to preliminary mechanical treatment and water washing with detergents (soap solution, washing powder). After thorough rinsing with tap water, immersed in a cuvette instruments, filled with a solution of methylene blue. Cuvette with the tools installed in the camera setup on a rotating pan. Unit door tightly closed, the timer is set exposure time include monochromatic light emitter is mounted above the cuvette, and the motor rotation pan. The process of inactivation of viruses on the surface of medical instruments continued for 45 minutes while processing monochromatic light of wavelength 590 nm [description of the patent UZ Nº FAP 00464, IPC A61L2 / 08, publ. 29.05.2009].

However, the usage of known methods of DNA and RNA viruses inactivation on medical instruments are not also effective due to the increased energy intensity and structural complexity. In addition, given that the methylene blue (photochemical agent) is capable of providing maximum activation only in the interaction with monochromatic light of wavelength 590nm, current method does not disclose the terms of the organization of the required coverage.

The basis of the invention is to develop an efficient, with minimal labor and energy consumption method for inactivating RNA and DNA viruses on medical instruments, by achieving maximum activation of the photochemical agent in the interaction with monochromatic light.

This aim is achieved by the method of inactivation of RNA and DNA viruses on medical instruments including preliminary mechanical cleaning and washing tools, their subsequent exposure in an aqueous solution of methylene blue with a maximum activation of the interaction of monochromatic light with a solution in which the invention maximal activation in aqueous 0.01 -0.02% solution of methylene blue concentration is effected by interaction with light in the spectrum of monochromatic radiation emitters with wavelength range of 658-662nm and 582-592nm or tools kept in the solution for 90 minutes.

The essence of the claimed process is that methylene blue solution is radiated by monochromatic light flux with wavelength 590nm or 660nm that corresponds to the wavelength of the absorption spectrum of methylene blue. For this purpose, a special source of radiation in the emission spectrum monochromatic emitters with a wavelength range of 582-592 nm and accordingly the maximum specific content of a monochromatic beam at 590 nn wavelength and the emission spectrum monochromatic emitters with a wavelength range 65 8-662 nm and the specific content of monochromatic beam with maximum wavelength of 660 nm. The appropriateness of methylene blue wavelength and the wavelength of monochromatic light flux is photoactivation of methylene blue. When there is photoactivation quantum energy molecules of methylene blue are absorbed and electrons excite in their atoms. As a result, the molecules themselves become sources of scattered light of the same wavelength (classical scattering). Under the influence of light quanta emitted by the photoactivated molecules of methylene blue there is photoactivation of molecules which are in the "shadow" relating to monochromatic radiator installation. Thus, to install all subjected to photoactivation of methylene blue molecule - as in the path of light rays emitter, and are in the "shadow" relative to it. Inactivation of RNA and DNA viruses for medical instruments is carried out at room temperature photoactivated liquid - in the 0.01-0.02% solution of methylene blue (Methylene Blue). 0.01-0.02% methylene blue solution was subjected to monochromatic light flux wavelength 590 nm or 660 nm that corresponds to the wavelength of the absorption spectrum of methylene blue. In setting a monochromatic light beam wavelength is from 660 nm to 590 nm, or a monochromatic emitter is created. Total energy consumption of device is 50 W, the light output must be at least 280 lm. Under the influence of monochromatic light flux wavelength of 590 nm or 660 nm (corresponding to the wavelength of the absorption spectrum of methylene blue) occurs photoactivation of methylene blue molecules. Photoactivated molecules of methylene blue actively enter into a strong bond with a pair of nucleic acids - guanine and cytosine and locks them in the chain of RNA or DNA viruses. Viruses with such inactivated RNA and DNA even when entering a person's blood are not capable of destroying cells and replicate inside cells. It means that they completely lose their virulence and pathogenic properties (ability to infect and cause disease). Moreover, the photoactivated molecules of methylene blue promote the formation of reactive atomic oxygen, which strengthens denaturation process of nucleic acids of RNA and DNA viruses.

The method is realized as follows.

Medical instruments (dental, gynecological, surgical, ophthalmic and others), after being used in patients, are subjected to preliminary mechanical cleaning and washing in water with detergents (soapy water, detergents). Thereafter, instruments are rinsed thoroughly in tap water and then immersed in a round cuvette device in 0.01-0.02% solution of methylene blue. Tools are put on the bottom of the cell stack in a single layer so that they are completely covered with a solution of methylene blue. Then, the cell with the tools mounted on the base of the camera unit. Unit door tightly closed, the timer is set to the selected exposure time toggle switch. At the same time the door is securely closed and till deactivation the timer should not be opened. When the device of the camera automatically turns on the panel radiator monochromatic light mounted above the cuvette. The process of inactivation of viruses present on the surface of medical instruments.

After switching off the timer the device board's door is opened, cell with tools is extracted. Tools are removed from the solution with tweezers and methylene blue is rinsed thoroughly in distilled water. Thereafter, conventional tools are further sterilized.

The photoactivatable fluid to inactivate DNA or RNA viruses is a 0.01-0.02% solution of methylene blue (Methylene Blue) in distilled water. 0.01-0.02% solution photoactivated liquid prepared by dissolving Methylene Blue, respectively 1,0-2,0g in 10 liters of distilled water in a clean container.

Preservation 0.01-0.02% methylene blue solution in a clean container is 10 days. Portion of 0.01-0.02% solution of methylene blue is suitable for 3-fold use within 1 day. Visually, the loss of transparency (turbidity) or plaque formation on the surface of a solution of methylene blue is considered unsuitable for virus inactivation. In this case the solution is prepared anew.

The closest to the essential features of the claimed plant can be taken to inactivate RNA and DNA viruses on medical instruments. This device comprising a flat-bottomed cell fluid and located above the radiation source according to the invention is provided with means for rotating the cell, means for fluid turbulence, and a battery of radiators monochromatic visible light to inactivate liquid having photoactive properties. The process of inactivation of viruses present on the surface of medical instruments continues for 45 minutes while processing monochromatic light of wavelength 590 nm [description of the patent UZ Nº FAP 00464, IPC A61L2 / 08, publ. 29.05.2009].

However, the use of known systems for inactivation of RNA and DNA viruses on medical instruments is not effective due to the increased energy intensity and structural complexity. In addition, methylene blue (photochemical agent) is capable of providing maximum activation of the interaction with monochromatic light of wavelength 590 nm, to a certain device conditions required lighting isn't disclosed.

The basis of the invention is to develop a simplified design, with minimal labor and energy expenditures for the inactivation of RNA and DNA viruses on medical instruments, by achieving maximum activation of the photochemical agent in the interaction with monochromatic light.

The problem is solved by the fact that the setting for the inactivation of RNA and DNA viruses on medical instruments, comprising a hermetically sealed housing with a camera, housed in a chamber container for tools, filled with a solution of methylene blue and installed capacity of monochromatic light source according to the invention monochromatic light source arranged in the emission spectrum monochromatic emitters with a wavelength range of 582-592 nn and 658-662 nm, with a total capacity of not less luminous flux 280 lm. As a monochromatic emitters LEDs were selected.

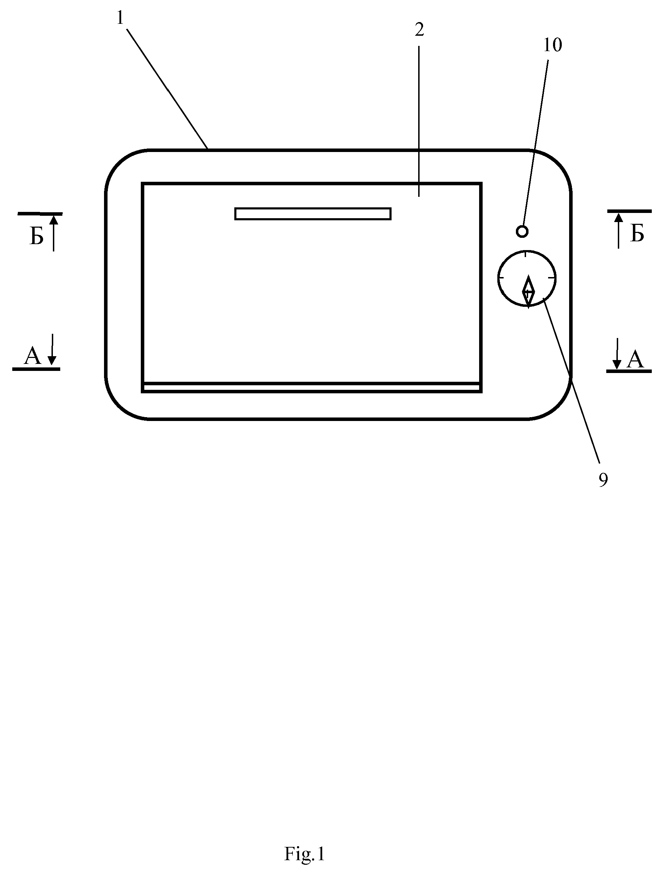

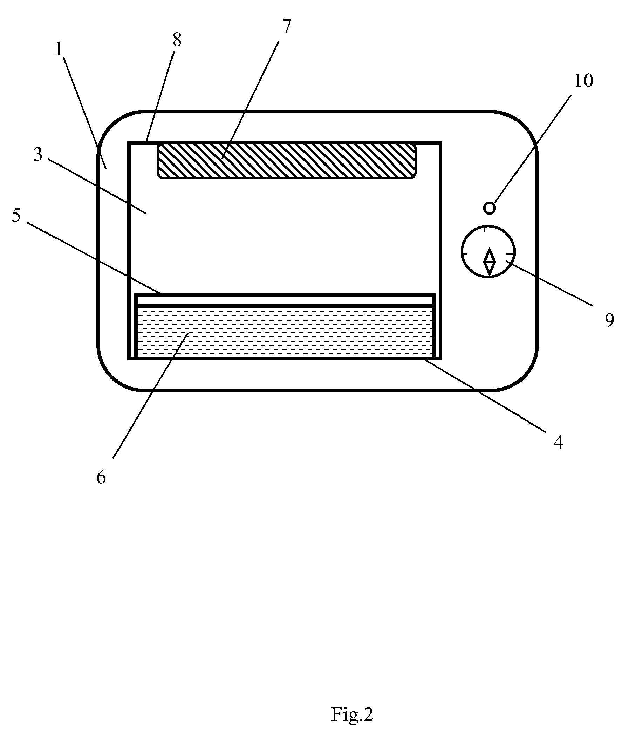

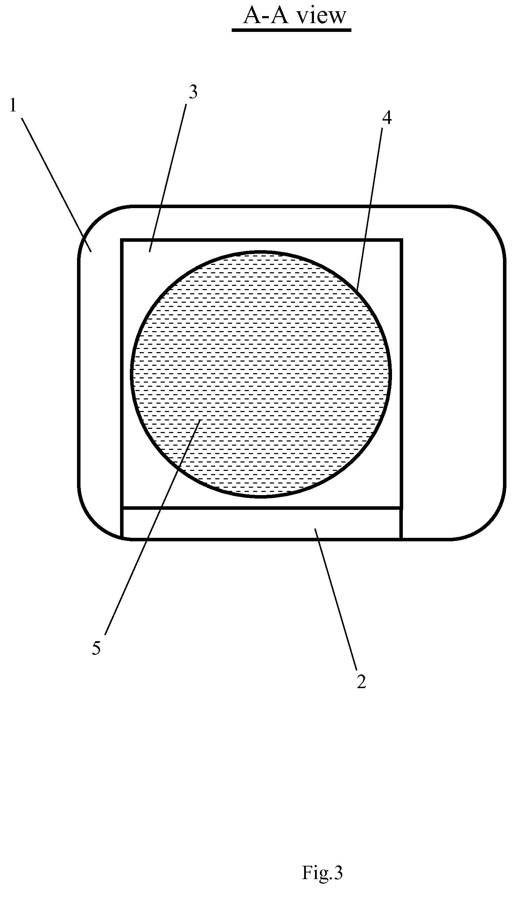

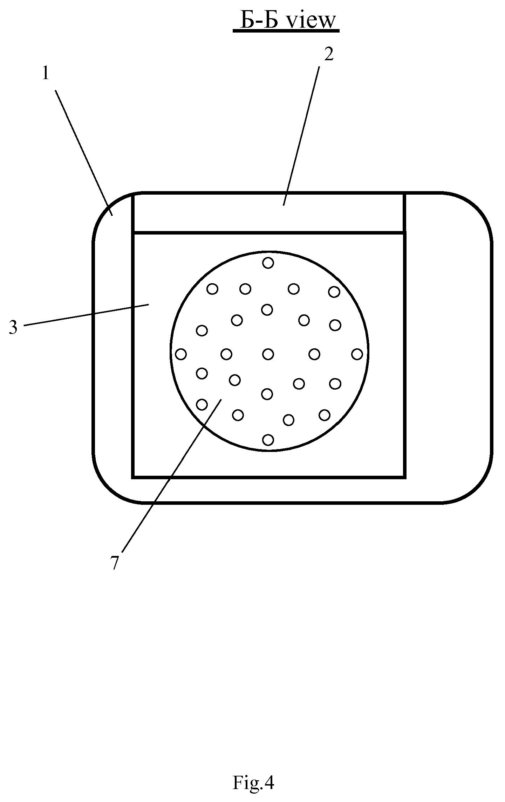

The device is schematically illustrated by the drawings Figure 1, 2, 3, 4.

Device 1 comprises a housing 2 with a door, ventilation holes in the sides (not shown) and the inner chamber 3. On the lower base of the chamber 4 is set to 5 cuvette photoactivated liquid (aqueous solution of methylene blue) 6. emitter monochromatic light 7 is mounted to the upper base 8 camera. Exposure time by timer 9, each division of the scale which corresponds to 15 minutes, the total time given by the timer is 90 minutes. The inclusion of a monochromatic emitter carried out by turning the timer knob in a clockwise direction with the door closed. When the set exposure time is off it is done automatically. The work of installing signal indicator light 10.

In the case of premature opening of the door locking device is activated and a monochromatic emitter is switched off.

The device operates as follows.

Medical instruments 6 are placed in a cuvette with an aqueous solution of methylene blue 7, which is subjected to irradiation of light energy monochromatic radiation with a maximum specific content of monochromatic light with a wavelength of 590 nm or 660 nm with a total light output of not less than 280 lm. The exposure time is set to 90 minutes. After treatment in the device the viruses contained in the medical instruments will be completely inactivated - lose the ability to infect cells of the human body. Instruments are reused without risk of infecting patients.

Monochromatic light emitter 8 with a maximum specific content of a monochromatic beam with a wavelength of 590 nm or 660 nm with a total capacity of not less luminous flux 280 lm, made in the form of LEDs, evenly placed on the panel, calibrated in the emission spectrum with a wavelength range of 582-592 nm or 658- 662nm.

Verification steps of the claimed method and installation is carried out on the biological environment contaminated by viruses, such as blood plasma.

### Example 1.

Monochromatic radiation emitters with a range of 590 ± 2 nm. After treatment in the inventive installation method claimed HBV containing plasma samples for 90 minutes in a 0.01% solution of methylene blue content of lymphocytes by PCR HBV DNA particles were not found. This indicates that after inactivation HBV completely lost the ability to penetrate the human lymphocytes.

In case of using monochromatic radiation emitter with a range of 588-592 nm reached full effect on HBV inactivation in 0.01% methylene blue solution.

### Example 2.

Monochromatic radiation emitters with a range of 590 ± 2 nm. After treatment in the inventive installation method HBV containing plasma samples for 90 minutes stay in a 0.02% solution of methylene blue content of lymphocytes by PCR HBV DNA particles were not found. This indicates that after inactivation HBV completely losts the ability to penetrate the human lymphocytes.

That means that, when using monochromatic radiation emitter with a range of 588 - 592 nm full effect of HBV inactivation in 0.02% methylene blue solution was reached.

### Example 3.

Monochromatic radiation emitters with a range of 660 ± 2 nm. After treatment in the inventive installation method claimed HBV containing plasma samples for 90 minutes in a 0.01% solution of methylene blue content of lymphocytes by PCR HBV DNA particles were not found. This indicates that after inactivation HBV completely lost the ability to penetrate the human lymphocytes.

In case of using monochromatic radiation emitter with a range of 658 - 662 nm was reached full effect on inactivating HBV inactivation in 0.01% methylene blue solution.

### Example 4.

Monochromatic radiation emitters with a range of 660 ± 2 nm. After treatment in the inventive installation method claimed HBV containing plasma samples for 90 minutes stays in a 0.02% solution of methylene blue, content of lymphocytes by PCR HBV DNA particles are not found.

This indicates that after inactivation HBV completely lost the ability to penetrate the human lymphocytes.

In case of using monochromatic radiation emitter with a range of 658 - 662 nm full effect on HBV inactivation in 0.02% methylene blue solution was reached.

Thus, the use of a claimed invention can effectively and cost-effectively achieve complete inactivation of RNA and DNA viruses on medical instruments and can be offered for a wide practical use in health care facilities.

## Claims

1. A method for inactivating RNA and DNA containing viruses on medical instruments comprising mechanical pre-purification and washing of tools, its further exposure to methylene blue aqueous solution with a maximum activation by reacting the solution with a monochromatic light, **characterized in that** the maximal activation in aqueous solution 0.01-0.02% methylene blue concentration is effected by interaction with light in the spectrum of monochromatic radiation emitters with wavelength range of 582-592 nn or 658-662 nm, and total light output not less than 280lm, wherein the tools kept in the solution for 90 minutes.

2. A device for inactivating RNA and DNA containing viruses on medical instruments, comprising a hermetically sealed housing with a camera, the camera is housed in a container for tools, filled with a solution of methylene blue and mounted above the capacity of a monochromatic light source, wherein the monochromatic light source comprises a monochromatic emitters in the emission spectrum with a wavelength range of 582-592nm and 658-662nm and a total capacity of luminous flux is not less than 280lm.

3. The device for inactivation of RNA and DNA viruses on medical instruments as claimed in claims 1-2, **characterized in that** the LEDs are chosen as a monochromatic emitters.
